# EUROPEAN PATENT SPECIFICATION

(11) **EP 1 534 249 B1**
(45) Date of publication and mention of the grant of the patent: **19.09.2012**
(21) Application number: 03741881.1
(22) Date of filing: 06.06.2003
(51) Int. Cl.: A61K 9/48, A61K 9/64, A61K 9/66

(54) **CHEWABLE SOFT CAPSULE**
WEICHE KAUKAPSEL
CAPSULE MOLLE A MACHER

(30) Priority: 07.06.2002 JP 2002167041
(43) Date of publication of application: 01.06.2005
(73) Proprietor: R.P. Scherer Technologies, LLC, Carson City, NV 89706 (US)
(72) Inventor: MAKINO, Hirokazu, Postal Code 436-0026 (JP); SUMINO, Yoko, Postal Code 436-0028 (JP); SUZUKI, Hideo, Post Code 436-0034 (JP); MASTUSHITA, Tomohisa, Post Code 436-0086 (JP)
(74) Representative: Dunleavy, Kevin James
(86) International application number: PCT/US2003/017746
(87) International publication number: WO 2003/103639

(56) References cited:
- WO-A-00/51574
- US-A- 2 580 683
- US-A- 4 935 243
- US-A- 4 935 243
- US-A- 5 554 385
- US-B1- 6 280 767
- US-B1- 6 319 518
- YOSHIMURA, K., TERASHIMA, M., HOZAN, D., EBATO, T., NOMURA, Y., ISHII, Y., AND SHIRAI, K.: "Physical properties of shark gelatin compared with pig gelatin" J. AGRIC. FOOD. CHEM., vol. 48, June 2000 (2000-06), pages 2023-2027, XP002509407
- ANONYMOUS: "Fish gelatin" INTERNET ARTICLE, [Online] 10 January 2001 (2001-01-10), XP002509408 Retrieved from the Internet: URL:http://web.archive.org/web/20010110084 000/http://www.norlandprod.com/techrpts/fi shgelrpt.html> [retrieved on 2009-01-08]

## Description

### TECHNICAL FIELD

The instant invention relates to soft gelatin capsule agents and capsules, particularly to an agent for making soft gelatin capsules and a dusted soft fish gelatin capsule for encapsulating medication or other consumables, exhibiting excellent texture and chewability qualities, as well as excellent storage qualities.

### BACKGROUND OF THE INVENTION

Experience has long shown that pharmaceuticals or other items for human or animal consumption may be safely packaged in a hard or soft gelatin shell. Gelatin is a substantially pure protein food ingredient, obtained by the thermal denaturation of collagen, which is the most common structural material and most common protein in animals. Gelatin forms thermally reversible gels with water, which gives gelatin products unique properties, such as reversible sol-gel transition states at near physiologic temperatures.

Gelatin is an amphoteric protein with an isoionic point between 5 and 9, depending on raw material and method of manufacture. Type A gelatin, with an isoionic point of 7 to 9, is derived from collagen with acid pretreatment. Type B gelatin, with an isoionic point of 4.8 to 5.2, is the result of alkaline pretreatment of the collagen. Like its parent protein collagen, gelatin is unique in that in contains, approximately, 16 % proline, 26 % glycine, and 18% nitrogen. Gelatin is not a complete protein food because the essential amino acid tryptophan is missing and the amino acid methionine is present only at a low level.

There are a large number of processes used in the manufacture of gelatin and the raw materials from which it is derived, including demineralized bone, pigskin, cow hide and fish. The proteinaceous material, collagen, and hence gelatin, can be derived from any edible protein containing material. For reasons of economy, gelatin can be most practically be derived from protein sources which would normally require refining before consumption and which would otherwise make up protein-containing waste material destined for animal feeds, agricultural fertilizers, or for other industries. However, in many cultures and areas of the world, gelatin processed form mammalian origins, that is, from beef or pigs, is not acceptable.

In the fish industry, there is considerable and unavoidable waste of fish protein, especially from the fish skins that remain after processing. The fish skin which remains after processing, especially filleting, is generally inedible as such, but can be used in the glue industry or for the manufacture of animal foodstuffs, fertilizers or other commodities of low commercial value.

However, fish skins have become a vital commercial source of gelatin. In general, the fish collagen is acidified to about pH 4 and then heated stepwise from 50° C to boiling to denature and solubilize the collagen. Then, the denatured collagen or gelatin solution has to be defatted, filtered to high clarity, concentrated by vacuum evaporation or membrane ultra-filtration treatment to a fairly high concentration for gelation, and dried by passing dry air over the gel. Finally, the dried gelatin is ground and processed into powder. The resulting gelatin has an isoionic point of 7 to 9 based on the severity and duration of the acid processing of the collagen which causes limited hydrolysis of the asparagine and glutamine amino acid side chains. Pharmaceutical and other agents can be encapsulated in either a hard or soft gelatin shell. Hard gelatin capsules are filled with dry materials such as powders or time-release beadlets by introducing the material into one section of a capsule and capping it with a second section.

Gelatin capsules may be classified as hard or soft, with a sub-classification, that of chewable capsules, within the soft class. Various plasticizing and hardening agents are added to the gelatin used to make capsules or microcapsules. The shell of a hard capsule is not plasticized, while a soft gelatin capsule is plasticized, often with Glycerol (glycerin), a plasticizer that is very widely used to make soft gelatin capsules. Other plasticizers used with, or instead of, glycerol include various alcohols, propylene glycol, sucrose, and acacia. Sorbitol is the most widely used alcohol, but other alcohols have been explored, including various polyethylene glycols (PEGs), mannitol, ethylene glycol, and tetrafurfuryl alcohol. Various starches can be used as disintegrants, to promote break-up of the capsule, and to improve adhesion of a secondary coating. Hard capsules may use aldehydes to cross-link and stiffen the structure of gelatin.

For human consumption, hard capsules are designed to be swallowed with dissolution of the capsule and absorption of the capsule contents in the gastrointestinal tract. While gelatins for the manufacture of hard gelatin capsules were traditionally obtained from mammalian tissues, U.S. Pat. App. Pub. No. 2001/0024678 details the manufacture of hard capsules from fish gelatin by means of adding a setting system comprising a hydrocolloid or mixtures of hydrocolloids and cations which may contain additional sequestering agents.

Soft gelatin capsules generally consist of a gelatin shell which is produced by casting a mixture of gelatin, plasticizer, and water into a thin sheet. The shell of a soft gelatin capsule is typically produced by adding, to a gelatin, a plasticizer in an amount of 30-40 wt % with respect to the gelatin, and drying the shell until the water content becomes 5-10 wt % so as to prevent the capsule from being deformed or becoming undesirably sticky. In some typical formulations, as seen in U.S. Pat. No. 5,554,385 to Stroud, a portion of the gelatin is replaced with a high amylose starch to provide a dry capsule sheath.

The soft gelatin capsule of such a formulation is relatively tough for optimal storage and handling, and is intended to dissolve after reaching the intestines so as to release its contents therein. Therefore, the capsule is not easily broken in the mouth and is not suitable for chewing.

Accordingly, while such gelatin capsules are comestible, they are not "edible" as the term is commonly used, to denote a material that is chewable or dissolvable in the mouth without unpleasant taste or texture sensations. The soft gelatin based compositions commonly employed to form the shells of soft gelatin capsules often yield a gummy, unpleasant, intractable mass in the mouth. However, a gelatin shell which is chewable and edible in the sense that it is pleasant tasting and can be readily fragmented, dissolved, and swallowed, would be advantageous from a number of perspectives.

For example, a person who is in medical distress from the sudden attack of a condition such as angina pectoris could achieve rapid release of the active ingredients of a capsule into the body with a truly chewable capsule. Ideally, the shell of the capsule would dissolve rapidly, without leaving any intractable, insoluble residue upon which the user might choke. In other applications, a chewable capsule can provide a convenient dosage form. Children, elderly, and other users often have difficulty swallowing pills or capsules, particularly without supplemental water to drink. In non-medical applications, a chewable capsule could contain, by way of example and not limitation, confectionary or breath freshening products in an easy to carry and use form.

Different routes have been used in the art to achieve the goals of a truly edible chewable gelatin capsule. For example, it is well know in the art, as described in U.S. Pat. No. 6,280,767 to Sano, et al. that increasing the amount of plasticizer, will increase the softness of the capsule. However, increasing plasticizer content is associated with an increased likelihood of a capsule sticking to another soft gelatin capsule or to a container, thereby causing deterioration in storage stability and damaged product. This is particularly true in high-temperature, high-humidity areas. Additionally, an increase in stickiness caused by increased plasticizer content creates a capsule that is more likely to stick to the teeth during mastication, an unpleasant tactile experience.

To compensate for the increased stickiness of high plasticizer formulations, one approach, seen in the '767 patent, is to supply a water insoluble cellulose in the capsule formulation. However, this leads to the unpleasant production of an insoluble residue in the mouth after chewing.

Another means to increase the softness and dissolution characteristics of a soft gelatin capsule is to increase the water content of the capsule, as seen in U.S. Pat. No. 4.,935,243 to Borkan, et al. In contrast to the conventional gelatin capsule with a water content of about 7%, Borkan et al. teaches a water content of 15-30 wt %. Higher water content is also employed in U.S. Pat. No. 2,580,683 to Krueger, which discloses capsule shells of gelatin, sugar and a minimum of about 34% water. A shell composition of about 45% water is also disclosed by Krueger '683. While increasing water enables a decrease in the amount of plasticizer employed, for example to approximately 25% in the '243 patent, high water content can result in capsule deformation, stickiness and storage problems.

International patent application publication no. WO 00/51574 discloses Chewable Soft gelatin capsules comprising two types of gelatin with low and high bloom strengths, plasticizer and an anti-adhesion agent.

Accordingly, the art has needed a chewable soft gelatin capsule that exhibits excellent texture and taste characteristics when chewed, does not leave an insoluble residue in the mouth, and has optimum moisture content and melting characteristics, and possesses excellent storage characteristics.

### SUMMARY OF THE INVENTION

The instant invention provides a novel agent for manufacturing a soft gelatin capsule, and for capsules made with the agent. The capsules have excellent "mouth feel", are easily dissolvable, and produce no residue when chewed. The capsules also perform well under storage conditions. The utilization of fish gelatin, is hoped to increase consumer acceptance in cultures that reject the use of mammalian gelatin.

In sum, the instant invention includes a soft gelatin capsule agent comprising a fish gelatin having a sol-gel transition temperature in a 10 wt.% aqueous solution of not more than 22° Centigrade and a sol-gel transition temperature in a 30 wt.% aqueous solution of not more than 32° Centigrade, a plasticizer, a partially pregelatinized starch, and an anti-adhesion agent. The most desirable sol-gel transition temperatures lay between 15° and 20° C for a 10% aqueous solution and between 25° and 30° C for a 30% aqueous solution. Sol-gel transition temperatures are determined by a water bath assay detailed below.

The plasticizer may be a polyol, particularly a polyol selected from the group consisting of glycerin, sorbitol, or mixtures thereof. One skilled in the art will realize that a number of plasticizers may be used in gelatin capsule formation, including by way of example and not limitation; polyethylene glycol, sucrose, mannitol, corn syrup, fructose, cellulose, dioctyl-sodium sulfosuccinate, triethyl citrate, tributyl citrate, 1,2-propylenglycol, mono-, di- or triacetates of glycerol, natural gums or the like as well as mixtures thereof. The anti-adhesion agent typically contains at least one starch, and the at least one starch may be corn starch. The capsule may be formulated with varying amounts of water and colorants.

In a preferred embodiment, the capsule agent comprises a mixture of 100 parts by weight of the selected gelatin, comprises between 100 and 130 parts by weight of plasticizer, and between 10 and 45 parts by weight of anti-adhesion agent. In another preferred embodiment, the mixture contains partially pregelatinized starch comprising 10 to 30 parts by weight of the mixture.

Optimally, the resulting capsule comprises a soft gelatin shell which comprises between about 36 wt.% and about 47 wt.% gelatin having a sol-gel transition temperature in a 10 wt.% aqueous solution of not more than 22° Centigrade and a sol-gel transition temperature in a 30 wt.% aqueous solution of not more than 32° Centigrade, about 47 wt.% plasticizer, and between about 4 wt.% and about 16 wt.% anti-adhesion agent; and a soft gelatin capsule fill material. The fill material may be selected from a near limitless array of foodstuffs, medicaments, and other substances.

The shell is typically formulated with water, which comprises between about 8 wt.% and about 25 wt.% of the soft capsule shell. The capsule may further comprise a surface coating applied to the exterior of the soft gelatin shell to decrease surface stickiness. The surface coating typically includes at least one starch, which may be a potato or a corn starch.

### BRIEF DESCRIPTION OF THE DRAWINGS

Referring now to the drawings and figures:
FIG. 1 shows a graph of the data presented in Table 8, showing the results of subjective texture evaluation over a period of six months for capsules made according to the instant invention.
FIG. 2 shows a graph of the data presented in Table 10, showing the results of subjective texture evaluation over a period of four months for capsules made according to the instant invention.
FIG. 3 shows a graph of the data presented in Table 11, showing the results of subjective texture evaluation over a period of four months for capsules made according to the instant invention.

### DETAILED DESCRIPTION OF THE INVENTION

The art is well acquainted with the use of fish gelatin to form various types of hard and soft gelatin capsules. Initial experimentation sought to identify those gelatins, which in formulation with a plasticizer, starch, colorant, and sweetener, might have the desired qualities of a good mouth feel, firm chewiness without excessive hardness, excellent storage characteristics, and also be susceptible to mass production techniques for capsule manufacture. The glycerin used was Japan Food Additive Standard JFAS (Japan Food Additive Standard) 99.5 grade and the corn starch was Hohnen HS-7 high amylose corn starch.

Formulations were tested by compounding mixtures, as specified in Table 1, casting the material in a sheet 0.7-1 mm thick, and then dividing the sheet into units 1 cm square. A panel of tasters assessed the samples for seven parameters; softness, easy dissolving, elasticity, powder or granular texture, chewiness, saliva stimulation, and sol-gel nature (subjective feeling of the liquidity of the compound) on a scale wherein 0 represent neutral judgments, while negative values and positive values represented departures from a neutral value.

As summarized in Table 1, five commercially available mammalian (Nitta S#195A Acid Gel, Miyagi RP-600 Modified Gel, and Nitta SCP -5000 Collagen Peptide) and fish (Miyagi MPM Shark Fish Gel and Croda 200 B Fish Gel) gelatins provided reasonable performance to taste, but not to production parameters. Nitta S#195A Acid Gel is a mammalian derived gelatin produced from acid treated bovine bone, having a Bloom strength of 210-240. Miyagi RP-600 Modified Gel is a mammalian derived gelatin produced by the reaction of succinic anhydride and alkali treated bone gelation, having a Bloom strength of 180-200. Nitta SCP-5000 Collagen Peptide is a hydrolyte of mammalian derived gelatin produced from acid treated porcine skin. Among the fish gelatins, Miyagi RPM Shark Fish Gel is a shark derived gelatin having a Bloom strength of 110-140. Croda 200B Fish Gel is derived from fish and has a Bloom strength of 195-210. In the experimentation described below, the Croda 200B Fish Gel had a measured Bloom strength of 207.

Specifically, the gelatins tested above, exhibited adequate, although somewhat sticky texture and feel to consumers, were excessively soft and sticky for machine capsule formation, making them unlikely candidates for either mass production or prolonged storage, and therefore represented inadequate commercial solutions. The shark gel produced the best subjective texture to a taste panel, but experiments 3A and 3B, as seen in Table 1, utilizing Croda 200B Fish Gel, produced the most promising combination of subjective texture and commercial handling qualities.

**Table 1 (all samples 120 parts glycerin, 42 parts corn starch, 1 part TiO, 5 parts aspartame)**

| No. | Gelatins | | | | | Water |
|---|---|---|---|---|---|---|
| | Acid Gel | Modified Gel | Collagen Peptide | Fish Gel | Fish Gel | |
| | Nitta Acid Gel | Miyagi RP-600 | Nitta SCP-5000 | Miyagi MPM Shark | Croda 200B | |
| 1 | | 100 | | | | 63 |
| | | 100 | | | | 113 |
| | | 100 | | | | 90 |
| | | 100 | | | | 80 |
| | | 100 | | | | 80 |
| 2 | 90 | | 10 | | | 63 |
| | 90 | | 10 | | | 60 |
| | 90 | | 10 | | | 55 |
| | 90 | | 10 | | | 58 |
| | 90 | | 10 | | | 58 |
| 3 | | | | 100 | | 63 |
| | | | | 100 | | 131 |
| | | | | 100 | | 110 |
| | | | | 100 | | 100 |
| | | | | 100 | | 98 |
| 3A | | | | | 100 | 100 |
| | | | | | 100 | 63 |
| | | | | | 100 | 75 |
| | | | | | 100 | 81 |
| 3B | | | | | 100 | 80 |
| | | | | | 100 | 85 |

All assessed as adequate to taste assessment, but too sticky for machine production.

Accordingly, experimentation was directed to decreasing the soft and sticky nature of the gelatin mixtures by combining them with various plasticizers. Typical results are reported in Table 2. In the protocol reported, all samples consisted of an added 42 parts corn starch, 1 part titanium oxide (colorant) and 5 parts aspartame, to approximate the additives most likely to be included in a commercial product. Additional additives besides the JFAS 99.5 Glycerin and corn starch previously mentioned included Propylene Glycol manufactured to Japan Food Additive Standard (JFAS) by Asahi Chemical, and Xylitol manufactured by Towa Chemical Industry Ltd., and marketed as XylitXC.

Glycerin, as a plasticizer, alone and in combination with propylene glycol, and glycerin in combination with Xylitol, produced good dissolution. Attempts to utilize additional additives, such as cellulose (Asahi Kasei Abcel RC-N Crystalline Cellulose, composed of 80% crystalline cellulose, 10% karaya gum, and 10% dextrin), SiO₂ (JFAS grade; Fuji Chemical Cycolpege 720), and NaHCO₃ (JFAS grade; Asahi Kasei) failed to produce significant improvements in performance, as reported in Table 3. However, the addition of partially pregelatinized starch, which is known in the art to have good dissolution qualities, to a mixture of gelatin and glycerin improved mouth feel and produced good dissolution of the capsule agent at various water levels, as shown in Table 4. One particular type of partially pregelatinized starch, Asahi Kasei PCSD FW-40 partially pregelatinized starch made from wheat, performed well.

Due to the promising texture tasting performed earlier with fish gelatin, attention was directed to optimizing combinations of glycerin and partially pregelatinized starch, along with fish gelatin, particularly the Croda fish gelatin which was seen to produce good results. The results are reported in Table 5. It was believed that the addition of an additional anti-adhesion agent might result in improved performance.

**Table 2 - (all samples 42 parts corn starch, 1 part TiO, 5 parts aspartame)**

| | Acid Gel | Glycerin | Propylene Glycol | Xylitol | Water |
|---|---|---|---|---|---|
| No. | Nitta S#195A | JFAS 99.5 | Asahi | Towa XylitXC | |
| 4 | 100 | 60 | 60 | | 80 |
| | 100 | 60 | 60 | | 70 |
| | 100 | 60 | 60 | | 65 |
| 5 | 100 | 116 | 4 | | 80 |
| | 100 | 116 | 4 | | 63 |
| | 100 | 116 | 4 | | 78 |
| 6 | 100 | 90 | | | 68 |
| | 100 | 90 | | | 80 |
| | 100 | 90 | | | 83 |
| 7 | 100 | 90 | | 30 | 68 |
| | 100 | 90 | | 30 | 77 |
| | 100 | 90 | | 30 | 83 |
| 8 | 100 | 86 | 4 | | 63 |
| | 100 | 86 | 4 | | 85 |
| | 100 | 86 | 4 | | 65 |
| | 100 | 86 | 4 | | 75 |

**Table 3 - (all samples 42 parts corn starch, 1 part TiO, 5 parts aspartame)**

| No. | Gelatin | | Plasticizer | Additives | | | |
|---|---|---|---|---|---|---|---|
| | Acid Gel | Collagen Peptide | Glycerin | Cellulose Crystalline | SiO2 | NaHCO3 | Water |
| | Nitta S#195 A | Nitta SCP-5000 | JFAS 99.5 | Asahi Kasei Abcel RC-N81 | Fuji Silisia Cylopege 720 | Asahi Kasei | |
| 9 | 50 | 50 | 120 | 1.5 | | | 63 |
| 9A | 70 | 30 | 120 | 1.5 | | | 63 |
| | 70 | 30 | 120 | 1.5 | | | 68 |
| 11 | 100 | | 120 | | 5 | | 63 |
| | 100 | | 120 | | 5 | | 73 |
| 13 | 100 | | 44 | | | 1.7 | 63 |
| | 100 | | 120 | | | 1.7 | 63 |
| | 100 | | 120 | | | 1.7 | 73 |

**Table 4 - (all samples 1 part TiO, 5 parts aspartame) All observed to be easy to dissolve**

| | Acid | Plasticizer | Partial Pregelatinized Starch | | |
|---|---|---|---|---|---|
| No. | Nitta S#195A | JFAS 99.5 | Asahi Kasai PCSD FC-50 | Asahi Kasai PCSD FW-40 | Water |
| 15 | 100 | 120 | | 42 | 100 |
| 15A | 100 | 120 | | 42 | 100 |
| 15B | 100 | 120 | | 42 | 105 |
| 15C | 100 | 120 | | 42 | 125 |

**Table 5 - (all samples 1 part TiO, 5 parts aspartame)**

| | Gelatin | Plasticizer | | | |
|---|---|---|---|---|---|
| No. | Croda 200B | Glycerin JFAS 99.5 | Partial Pregelatinized Starch Asahi Kasei PCSD FW-40 | Water | Comments |
| 16 | 100 | 120 | 42 | 100 | Compared to 3A brittle and difficult to encapsulate |
| | 100 | 120 | 42 | 105 | |
| | 100 | 120 | 42 | 125 | |
| 17 | 100 | 100 | 42 | 115 | Compared to 16, sticky and judged encapsulation difficult |
| | 100 | 100 | 42 | 125 | |
| 18 | 100 | 80 | 42 | 130 | Compared to 17, sticky and judged encapsulation difficult |

The addition of additional starch, in the form of Hohnen HS-7 high amylose corn starch, along with the partially pregelatinized starch of the previous experiments, resulted in the optimal capsule formulations, as shown in Table 6. Additionally, it was discovered that a key characteristic necessary to create capsules of both good subjective mouth feel and mass production characteristics lay in the sol-gel temperature characteristics of the gelatin used.

Sol-gel and gel-sol transition temperatures were assayed according to the following protocols. To assess gel-sol (solid to liquid) transition temperatures, gelatin solutions are cast in a test tube and maintained well below physiologic transition temperatures, in this case at 10° C. A lead shot is placed on the surface of the firm gelatin, which due to its gel nature is capable of holding the weight of the shot. The test tube is placed in a water bath and gradually raised in temperature at the rate of 12° C per hour. The process is carefully observed while heating, and the gel-sol transition temperature, or melting point, is the temperature at which the gelatin liquefies sufficiently such that the shot drops to the bottom of the test tube.

Conversely, the sol-gel (liquid to solid) transition temperature is determined as follows. Gelatin solutions are placed in a test tube at temperatures well above physiologic transition temperature, in this case at 60° C. The test tube is placed in a water bath and gradually lowered in temperature at the rate of 12° C per hour. The process is carefully observed while cooling, and the sol-gel transition temperature, or setting point, is the temperature at which the gelatin hardens sufficiently such that an adherent droplet forms on a stirring rod immersed and then withdrawn from the solution.

Gelatin formulations with acceptable characteristics were found to have a relatively narrow range of acceptable sol-gel transition temperatures. That is, optimal subjective mouth feel and mass production characteristics occurred only using fish gelatin that displayed a sol-gel transition temperature, in a 10% aqueous solution, of less than 22° C (approx. 72° F), or when in a 30% aqueous solution, of less than 32° C (Approx. 90° F). The most desirable sol-gel transition temperatures lay between 15° and 20° C for a 10% aqueous solution and between 25° and 30° C for a 30% aqueous solution.

By asking tasters to quantify their subjective judgments using a numerical scale, it was hoped that a certain degree of consistency could be introduced to observations made over time. As a predicate to testing sample lots after various storage times under various storage conditions, tasters were asked to rate the subjective texture of several lots of experimental mixtures, formulated into both round and oval capsule shapes, from the successful formulation of Experiment 19 (see Table 6).

The gelatin capsule agent of Experiment 19 was viscosity adjusted to a viscosity of 9,000 mPa, plus or minus 2,000 mPa at 54° C (plus or minus 2°C) as tested on a Brookfield Type B viscometer with a No. 4 spindle at 12 rpm. The agent was then cast into ribbons with a thickness of 0.028-0.029 inches (0.071-0.074 cm) and formed by a standard rotary die process into capsules weighing approximately 113 mg (plus or minus 7 mg). The capsules were filled using standard pharmaceutical techniques with a mixture of fractionated coconut oil (medium chain fatty acid triglyceride), mint flavoring, and aspartame sweetener. As with the previous experiments using sheet gelatin formulation samples, a panel of tasters assessed the capsules according to seven parameters; softness, easy dissolving, elasticity, powder or granular texture, chewiness, saliva stimulation, and sol-gel nature (subjective feeling of the liquidity of the compound) on a scale wherein 0 represents neutral judgments, while negative values and positive values represented departures from a neutral value. The results are shown in Table 7.

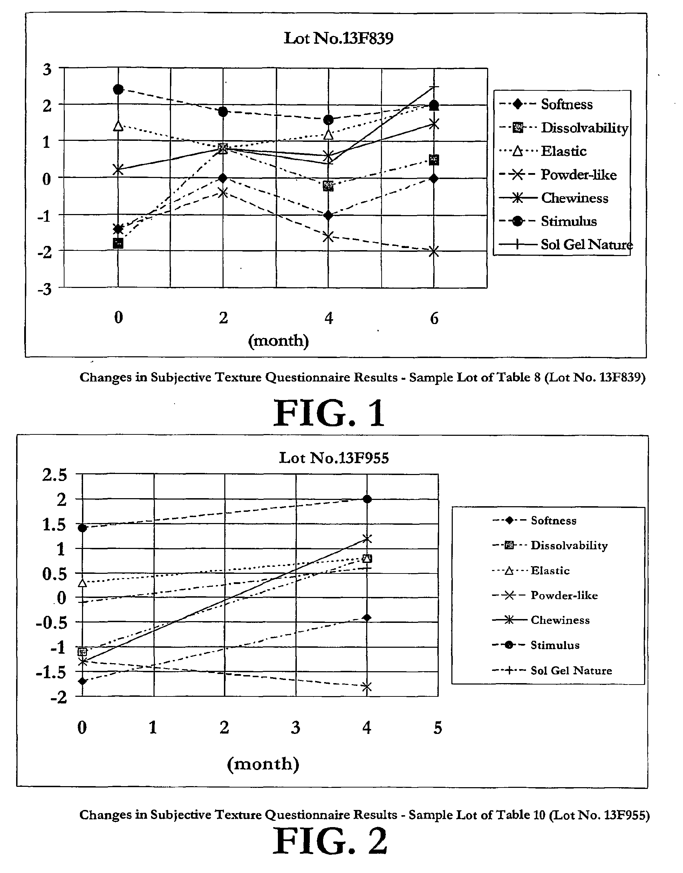

A test lot of capsules from the same formulation was then placed into storage at room temperature (approximately 15°-25° C), and evaluated by tasters at one, two, four, and six months after manufacture. To the degree practical, tasters remained the same throughout the experiment. The results are reported in tabular form in Table 8, and in graphic form in FIG. 1.

As seen in FIG. 1, there was considerable stability reported over the entire six month period of the experiment. There was a slight increase noted in hardness over time. Also, over time, the capsules were perceived as being more gelled and less liquid in nature.

**Table 6 - Formulations of the Instant Invention Experimental Formulation No. 19 Selected for Stability Testing**

| | Fish Gel | Glycerin Plasticizer | Partial Pregelatinized Starch | Corn Starch | | |
|---|---|---|---|---|---|---|
| No. | Croda 200B | JFAS 99.5 | Asahi Kasei PCSD FW-40 | Hohnen HS-7 | Water | Comments |
| 19 | 100 | 120 | 20 | 22 | 100 | Good stretch, soft good dissolution in mouth. |
| | 100 | 120 | 20 | 22 | 110 | |
| | 100 | 120 | 20 | 22 | 110 | |
| | 100 | 120 | 20 | 22 | 112 | |
| | 100 | 120 | 20 | 22 | 112 | |
| 20 | 100 | 120 | 10 | 32 | 100 | Good in dissolution |
| | 100 | 120 | 10 | 32 | 105 | |
| 21 | 100 | 120 | 30 | 12 | 100 | Good |
| | 100 | 120 | 30 | 12 | 120 | |
| | 100 | 120 | 30 | 12 | 130 | |

Samples were also placed in cold storage (approximately 4°-5° C) and removed for subjective testing at one and two months after manufacture. The results are reported at Table 9. Interestingly, compared to the results reported above in Table 8 and FIG. 1, the cold stored samples showed no less stability in most parameters than those that had been stored at room temperature (approximately 15°-25° C).

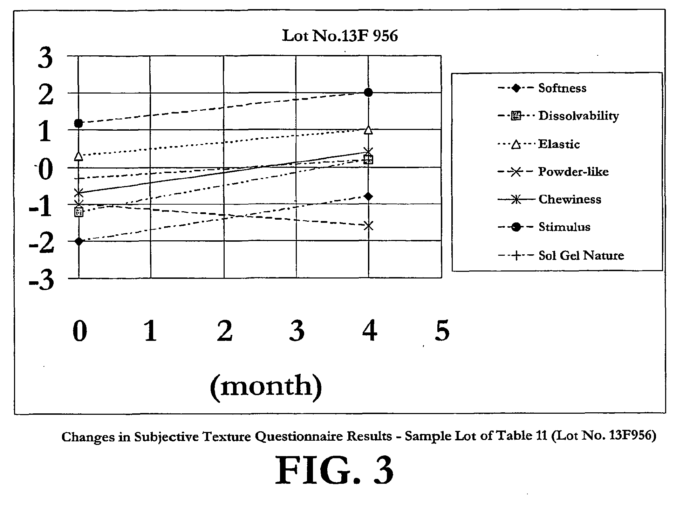

Additional testing was performed on two additional sample lots immediately following manufacture and after four months of storage at room temperature (approximately 15°-25° C). The first of these experiments is reported in Table 10 and FIG. 2; and the second experiment is reported in Table 11 and FIG. 3. Both experiments showed some increase in hardness and dissolvability over time, although this was judged within acceptable limits.

However, in the course of this experimentation, it was found that the residual moisture of the capsules created a tendency for them to deform and stick together during storage. Accordingly, a protocol was devised to reduce surface stickiness.

Capsules were manufactured using a rotary die process. The capsules were then tumble dried to remove water to a level of a moisture content typically in the range of 8-25%. For example experimental lot 13F839 (Tables 7, 8, and FIG. 1) had a residual shell water content of 16.0%, lot 13F840 (Table 7) had a residual shell water content of 13.4%, lot 13F955 (Tables 7, 10, and FIG. 2) had a residual shell water content of 14.9%, and lot 13F956 (Tables 7, 11, and FIG. 3) had a residual shell water content of 14.1%.

After the tumble dry process, the capsules were transferred into either a polishing pan or an automated inline dusting system whereby the product was coated with a layer of starch, typically potato or corn starch, although tapioca starch, wheat powder, waxy corn starch powder, and partial alpha starch powder were also effective. The capsules were then tumbled to produce an even coating of starch which prevented the capsules from sticking to one another.

After tumbling, the capsules were transferred onto a vibratory sieve where they were vibrated to remove excess coating material. The product was then bulk packaged.

Dusted capsules were compared with undusted controls to assess stickiness in storage under expected field conditions. Ten capsules were placed in a glass bottle and stored at 35° C for one week, followed by one day at 40° C. The capsule stickiness was observed by turning the bottle over and observing whether the capsules had adhered to each other or not. Capsules were evaluated without dusting and at levels of 0.05%, 0.1%, 0.2%, 0.5%, 1.0%, and 2.0% starch by weight of the capsules. The dusted starch percentage weights is calculated as the amount of starch that is added to a particular lot of capsules, rather than the amount of starch which actually adheres to each capsule. In one exemplary test lot, 25 g of powder was added to a lot of 15,000 capsules weighing 5 kg, producing a percentage weight of 0.5%. While there was significant stickiness of the capsules without starch, a starch level of about 0.5% provided optimal results in preventing capsule sticking.

**Table 7 -Average Values of Initial Subjective Texture Questionnaires Experimental Formulation No. 19**

| Lot No. | 13F839 | 13F840 | 13F955 | 13F956 |
|---|---|---|---|---|
| Size | 2 oval | 2 round | 3 round | 4 round |
| Time Elapsed Since Manufacture Prior to Testing | 1 Month | 2 Months | Immediate | Immediate |
| Softness | 1.4 | 0.4 | 1.7 | 2.0 |
| Dissolvability | 1.8 | 0.3 | 1.1 | 1.2 |
| Elastic | 1.4 | 0.1 | 0.3 | 0.3 |
| Powder-like | 1.4 | 1.1 | 1.3 | 1.0 |
| Chewing | 0.2 | 0.3 | 1.3 | 0.7 |
| Saliva Stimulus | 2.4 | 1.3 | 1.4 | 1.2 |
| Gelling | 0.0 | 1.6 | 0.1 | 0.3 |

| | |
|---|---|
| Croda 200B Fish Gel | 100 parts/wt. |
| JFAS 99.5 Glycerin | 120 parts/wt |
| Asahi Kasei PCSD FW-40 Partially Pregelatinized Starch | |
| Hohnen HS-7 Corn Starch | 22 parts/wt |

**Table 8 - Subjective Texture Questionnaire Results; Sample Lot 13F839; 0 to 6 Month Time**

| Lot No.13F839 | | |
|---|---|---|
| | | Room Temp. 1 Month |
| | | Average |
| ① | Softness | -1.4 |
| ② | Dissolvability | -1.8 |
| ③ | Elastic | 1.4 |
| ④ | Powder-like | -1.4 |
| ⑤ | Chewing | -0.2 |
| ⑥ | Saliva Stimulus | 2.4 |
| ⑦ | Gelling of fill material | - |

| | | Room Temp. 2 Months (10/2/01) |
|---|---|---|
| | | Average |
| ① | Softness | 0.0 |
| ② | Dissolvability | 0.8 |
| ③ | Elastic | 0.8 |
| ④ | Powder-like | -0.4 |
| ⑤ | Chewing | 0.8 |
| ⑥ | Saliva Stimulus | 1.8 |
| ⑦ | Gelling of fill material | 0.8 |

| | | Room Temp. 4 Months |
|---|---|---|
| | | Average |
| ① | Softness | -1.0 |
| ② | Dissolvability | -0.2 |
| ③ | Elastic | 1.2 |
| ④ | Powder-like | -1.6 |
| ⑤ | Chewing | 0.6 |
| ⑥ | Saliva Stimulus | 1.6 |
| ⑦ | Gelling of fill material | 0.4 |

| | | Room Temp.6 Months |
|---|---|---|
| | | Average |
| ① | Softness | 0.0 |
| ② | Dissolvability | 0.5 |
| ③ | Elastic | 2.0 |
| ④ | Powder-like | -2.0 |
| ⑤ | Chewing | 1.5 |
| ⑥ | Saliva Stimulus | 2.0 |
| ⑦ | Gelling of fill material | 2.5 |

**Table 9 - Change over Time in Subjective Texture Questionnaire Cold Storage (approximately 4°-5° C)**

| Cold Storage | |
|---|---|
| Lot No.13F839 | |
| | Room Temp. 1 Month |
| | Average Value |
| Softness | -1.4 |
| Dissolvability | -1.8 |
| Elastic | 1.4 |
| Powder-like | -1.4 |
| Chewing | -0.2 |
| Saliva Stimulus | 2.4 |
| Gelling of fill material | -- |

| | Cold Storage 2 Months |
|---|---|
| | Average Value |
| Softness | -0.4 |
| Dissolvability | 0.2 |
| Elastic | 0.8 |
| Powder-like | -0.4 |
| Chewing | 0.0 |
| Saliva Stimulus | 1.6 |
| Gelling of fill material | 0.6 |

**Table 10 - Change over Time in Subjective Texture Questionnaire Room Temperature (approximately 15°-25° C)**

| Changes on Storage | | |
|---|---|---|
| | Lot. No. 13F955 | Room Temp. 0 Months |
| | | Average Value |
| Softness | | -1.7 |
| Dissolvability | | -1.1 |
| Elastic | | 0.3 |
| Powder-like | | -1.3 |
| Chewing | | -1.3 |
| Saliva Stimulus | | 1.4 |
| Gelling of fill material | | -0.1 |

| | | Room Temp. 4 Months |
|---|---|---|
| | | Average Value |
| Softness | | |
| Dissolvability | | 0.8 |
| Elastic | | 0.8 |
| Powder-like | | -1.8 |
| Chewing | | 1.2 |
| Saliva Stimulus | | 2.0 |
| Gelling of fill material | | 0.6 |

**Table 11- Change over Time in Subjective Texture Questionnaire Room Temperature (approximately 15°-25° C)**

| Changes with Storage | | |
|---|---|---|
| Lot No.13F956 | Room Temp. 0 Months | |
| | Test Condition | Average Value |
| Softness | | -2.0 |
| Dissolvability | | -1.2 |
| Elastic | | 0.3 |
| Powder-like | | -1.0 |
| Chewing | | -0.7 |
| Saliva Stimulus | | 1.2 |
| Gelling of fill mataterial | | -0.3 |

| | Room Temp. 4 Months | |
|---|---|---|
| | Test Condition | Average Value |
| Softness | | -0.8 |
| Dissolvability | | 0.2 |
| Elastic | | 1.0 |
| Powder-like | | -1.6 |
| Chewing | | 0.4 |
| Saliva Stimulus | | 2.0 |
| Gelling of fill material | | 0.2 |

In sum, the instant invention provides a chewable soft gelatin capsule comprising a gelatin having a sol-gel transition temperature in a 10 wt.% aqueous solution of not more than 22° Centigrade and a sol-gel transition temperature in a 30 wt.% aqueous solution of not more than 32° Centigrade, a plasticizer, and an anti-adhesion agent. The most desirable sol-gel transition temperatures for preferred gelatin are between 15° and 20° C for a 10% by weight aqueous solution and between 25° and 30° C for a 30% by weight aqueous solution.

The gelatin is typically a fish gelatin, although other types of gelatin having the sol-gel temperature behavior of the instant invention are also intended. The plasticizer may also be, by way of example and not limitation, a polyol, particularly a polyol selected from the group consisting of glycerin, sorbitol, or mixtures thereof. One skilled in the art will realize that a number of plasticizers may be used in gelatin capsule formation, including by way of example and not limitation; polyethylene glycol, sucrose, mannitol, corn syrup, fructose, cellulose, dioctyl-sodium sulfosuccinate, triethyl citrate, tributyl citrate, 1,2-propylenglycol, mono-, di- or triacetates of glycerol, natural gums or the like as well as mixtures thereof.

The anti-adhesion agent typically contains at least one starch, and the at least one starch may be corn starch. The capsule may be formulated with varying amounts of water and colorants.

In a preferred embodiment, the capsule agent comprises a mixture of 100 parts by weight of the selected gelatin, between 100 and 130 parts by weight of plasticizer, and between 10 and 45 parts by weight of anti-adhesion agent. In another preferred embodiment, the mixture also contains partially pregelatinized starch, typically comprising 10 to 30 parts by weight of the mixture.

The soft gelatin capsule agent may be formulated into soft gelatin capsules by any of the means of manufacturing gelatin capsules that would be known to one skilled in the art. Optimally, the resulting capsule comprises a soft gelatin shell which further comprises between about 36 wt.% and about 47 wt.% gelatin having a sol-gel transition temperature in a 10 wt.% aqueous solution of not more than 22° Centigrade and a sol-gel transition temperature in a 30 wt.% aqueous solution of not more than 32° Centigrade, about 47 wt.% plasticizer, and between about 4 wt.% and about 16 wt.% anti-adhesion agent; and a soft gelatin capsule fill material. The most desirable sol-gel transition temperatures for gelatin are between 15° and 20° C for a 10% aqueous solution and between 25° and 30° C for a 30% by weight aqueous solution.

The soft gelatin fill material may be selected from a near limitless array of foodstuffs, medicaments, and other substances. The capsule maybe formulated with water, which typically comprises between about 8 wt.% and about 25 wt.% of the soft capsule shell.

The capsule may further comprise a surface coating applied to the exterior of the soft gelatin shell to decrease surface stickiness. The surface coating typically includes at least one starch, which may include a potato or a corn starch.

The soft gelatin capsules of the instant invention enable a significant advance in the state of the art. The preferred embodiments of the apparatus accomplish this by new and novel arrangements of elements that are configured in unique and novel ways and which demonstrate previously unavailable but preferred and desirable capabilities.

The detailed description set forth above in connection with the drawings is intended merely as a description of the presently preferred embodiments of the invention, and is not intended to represent the only form in which the present invention may be constructed or utilized. The description sets forth the designs, functions, means, and methods of implementing the invention in connection with the illustrated embodiments.

### INDUSTRIAL APPLICABILITY

The present invention answers a long felt need for a chewable soft gelatin capsule agent that exhibits pleasant mouth feel, good chewing characteristics, good storage characteristics, and is also susceptible to conventional mass production techniques. The capsules that are manufactured from this agent may be used to store a wide range of foodstuffs, medicaments, or other substances. The utilization, in some embodiments, of fish gelatin, is hoped to increase consumer acceptance in cultures that reject the use of gelatin produced from certain animal sources.

## Claims

1. A soft gelatin capsule agent including: a fish gelatin having a sol-gel transition temperature in a 10 wt.% aqueous solution of not more than 22 °C and a sol-gel transition temperature in a 30 wt.% aqueous solution of not more than 32 °C: a plasticizer; a partially pregelatinized starch and an anti-adhesion agent, wherein the sol-gel transition temperature is determined by placing a gelatin solution in a test tube at a temperature of 60°C, lowering the temperature of the test tube containing the gelatin solution at a rate of 12°C per hour and determining the temperature at which the gelatin hardens sufficiently such that an adherent droplet of the gelatin solution forms on a stirring rod immersed in and then withdrawn from the gelatin solution.

2. The capsule agent of claim 1, wherein the sol-gel transition temperature for a 10% aqueous solution of said gelatin is between 15 °C and 20°C and the sol-gel transition temperature is between 25 °C and 30 °C for a 30% aqueous solution of said gelatin.

3. The capsule agent of any one of claims 1-2, wherein the plasticizer is selected from one or more polyols.

4. The capsule agent of any one of claims 1-2, wherein the plasticizer is selected from glycerin, sorbitol, and mixtures thereof.

5. The capsule agent of any one of claims 1-4, wherein the anti-adhesion agent is at least one starch.

6. The capsule agent of claim 5, wherein the at least one starch is corn starch.

7. The capsule agent of any one of claims 1-6, further including water.

8. The capsule agent of any one of claims 1-7, further including a colorant.

9. The capsule agent of any one of claims 1-8, wherein, the gelatin makes up 100 parts by weight of the agent; the plasticizer makes up between 100 and 130 parts by weight of the agent; and the anti-adhesion agent makes up between 10 and 45 parts by weight of the agent.

10. A soft gelatin capsule agent as claimed in any one of claims 1-9, wherein the partially pregelatinized starch makes up between 10 and 30 parts by weight of the agent.

11. A soft gelatin capsule including a soft gelatin shell including the soft gelatin capsule agent of any one of claims 1-10, and a soft gelatin capsule fill material.

12. A soft gelatin capsule as claimed in claim 11, wherein the soft gelatin shell includes between 36 wt.% and 47 wt.% of said gelatin, 47 wt.% plasticizer, and between 4 wt.% and 16 wt.% anti-adhesion agent.

13. The capsule of any one of claims 11-12, having a water content of between 8 wt.% and 25 wt.%.

14. The capsule of any one of claims 11-13, further including a surface coating applied to the exterior of the soft gelatin shell to decrease surface stickiness, and wherein the surface coating includes at least one starch.

15. The capsule of claim 14, wherein the at least one starch in the surface coating is potato starch.

16. The capsule of claim 14, wherein the at least one starch in the surface coating is corn starch.

## Patentansprüche

1. Ein Weichgelatinekapsel-Wirkstoff umfassend: eine Fischgelatine mit einer Sol-Gel-Übergangstemperatur in einer wässrigen Lösung von 10 Gew.-% von nicht mehr als 22 °C und einer Sol-Gel-Übergangstemperatur in einer wässrigen Lösung von 30 Gew.-% von nicht mehr als 32 °C; einen Weichmacher; eine teilweise vorgelatinierte Stärke und einen Antiadhäsions-Wirkstoff, wobei zur Ermittlung der Sol-Gel-Übergangstemperatur eine Gelatinelösung bei einer Temperatur von 60 °C in ein Reagenzröhrchen platziert wird, die Temperatur des Reagenzröhrchens mit der Gelatinelösung um 12 °C pro Stunde gesenkt wird und die Temperatur bestimmt wird, bei der die Gelatine so ausreichend härtet, dass sich an einem in die Gelatinelösung eingetauchten und dann zurückgezogenen Rührstab ein anhaftender Tropfen der Gelatinelösung bildet.

2. Der Kapselwirkstoff gemäß Anspruch 1, wobei die Sol-Gel-Übergangstemperatur für eine 10-prozentige wässrige Lösung besagter Gelatine zwischen 15 °C und 20 °C liegt und die Sol-Gel-Übergangstemperatur für eine 30-prozentige wässrige Lösung besagter Gelatine zwischen 25 °C und 30 °C liegt.

3. Der Kapselwirkstoff gemäß irgendeinem der Ansprüche 1-2, wobei der Weichmacher unter einem oder mehreren Polyolen ausgewählt wird.

4. Der Kapselwirkstoff gemäß irgendeinem der Ansprüche 1-2, wobei der Weichmacher unter Glycerin, Sorbitol und Mischungen davon ausgewählt wird.

5. Der Kapselwirkstoff gemäß irgendeinem der Ansprüche 1-4, wobei der Antiadhäsions-Wirkstoff mindestens eine Stärke ist.

6. Der Kapselwirkstoff gemäß Anspruch 5, wobei die mindestens eine Stärke Maisstärke ist.

7. Der Kapselwirkstoff gemäß irgendeinem der Ansprüche 1-6, ferner Wasser umfassend.

8. Der Kapselwirkstoff gemäß irgendeinem der Ansprüche 1-7, ferner einen Farbstoff umfassend.

9. Der Kapselwirkstoff gemäß irgendeinem der Ansprüche 1-8, wobei die Gelatine 100 Gewichtsteile des Wirkstoffes ausmacht; der Weichmacher zwischen 100 und 130 Gewichtsteile des Wirkstoffes ausmacht; und der Antiadhäsions-Wirkstoff zwischen 10 und 45 Gewichtsteile des Wirkstoffes ausmacht.

10. Ein Weichgelatinekapsel-Wirkstoff gemäß irgendeinem der Ansprüche 1-9, wobei die teilweise vorgelatinierte Stärke zwischen 10 und 30 Gewichtsteile des Wirkstoffes ausmacht.

11. Eine Weichgelatinekapsel umfassend eine Weichgelatinehülle umfassend den Weichgelatinekapsel-Wirkstoff gemäß irgendeinem der Ansprüche 1-10 und ein Weichgelatinekapsel-Füllmaterial.

12. Ein Weichgelatinekapsel-Wirkstoff gemäß Anspruch 11, wobei die Weichgelatinehülle zwischen 36 Gew.-% und 47 Gew.-% der besagten Gelatine, 47 Gew.-% Weichmacher und zwischen 4 Gew.-% und 16 Gew.-% Antiadhäsions-Wirkstoff umfasst.

13. Die Kapsel gemäß irgendeinem der Ansprüche 11-12 mit einem Wassergehalt von zwischen 8 Gew.-% und 25 Gew.-%.

14. Die Kapsel gemäß irgendeinem der Ansprüche 11-13, ferner umfassend eine auf dem Äußeren der Weichgelatinehülle aufgebrachte Oberflächenbeschichtung zur Reduzierung der Oberflächenklebrigheit, wobei die Oberflächenbeschichtung mindestens eine Stärke umfasst.

15. Die Kapsel gemäß Anspruch 14, wobei die mindestens eine Stärke in der Oberflächenbeschichtung Kartoffelstärke ist.

16. Die Kapsel gemäß Anspruch 14, wobei die mindestens eine Stärke in der Oberflächenbeschichtung Maisstärke ist.

## Revendications

1. Agent de gélatine molle pour capsule comprenant : une gélatine de poisson ayant une température de transition sol-gel dans une solution aqueuse de 10 % pds qui ne dépasse pas 22 °C et une température de transition sol-gel dans une solution aqueuse de 30 % pds qui ne dépasse pas 32 °C; un plastifiant; un amidon partiellement pré-gélatinisé et un agent anti-adhésion, **caractérisée en ce que** la température de transition est déterminée en plaçant la solution de gélatine dans un tube à essai à 60 °C, en faisant baisser la température du tube à essai contenant la solution de gélatine de 12 °C par heure, et en déterminant la température à laquelle la gélatine se durcit suffisamment pour former une gouttelette de gélatine adhérente à une tige d'agitation, immergée, et ensuite retirée de la solution de gélatine.

2. Agent pour capsule de la revendication 1, **caractérisé en ce que** la température de transition sol-gel pour une solution aqueuse à 10 % de ladite gélatine est comprise entre 15 °C et 20 °C, et la température de transition sol-gel est comprise entre 25 °C et 30 °C pour une solution aqueuse à 30 % de la dite gélatine.

3. Agent pour capsule selon l'une quelconque des revendications 1 à 2, **caractérisé en ce que** le plastifiant se trouve sélectionné parmi un ou plusieurs polyols.

4. Agent pour capsule selon l'une quelconque des revendications 1 à 2, **caractérisé en ce que** le plastifiant se trouve sélectionné parmi la glycérine, le sorbitol et des mélanges de ceux-ci.

5. Agent pour capsule selon l'une quelconque des revendications 1 à 4, **caractérisé en ce que** l'agent anti-adhésion est au moins un amidon.

6. Agent pour capsule selon la revendication 5, **caractérisé en ce que** le au moins un amidon est un amidon de maïs.

7. Agent pour capsule selon l'une quelconque des revendications 1 à 6 comprenant en outre de l'eau.

8. Agent pour capsule selon l'un quelconque des revendications 1 à 7 comprenant en outre un colorant.

9. Agent pour capsule selon l'une quelconque des revendications 1 à 8, **caractérisé en ce que** la gélatine constitue 100 parties en poids de l'agent, le plastifiant constitue entre 100 et 130 parties en poids de l'agent, et l'agent anti-adhésion constitue entre 10 et 45 parties en poids de l'agent.

10. Agent de gélatine molle pour capsule selon l'une quelconque des revendications 1 à 9, **caractérisé en ce que** l'amidon partiellement pré-gélatinisé constitue entre 10 et 30 parties en poids de l'agent.

11. Capsule de gélatine molle comprenant une enveloppe de gélatine molle, comprenant l'agent de gélatine molle selon l'une quelconque des revendications 1 à 10, et une substance de remplissage de la capsule de gélatine molle.

12. Capsule de gélatine molle selon la revendication 11, **caractérisée en ce que** l'enveloppe de gélatine molle comprend entre 36 % pds et 47 % pds de la dite gélatine, 47 % pds du plastifiant, et entre 4 % pds et 16 % pds de l'agent anti-adhésion.

13. Capsule selon l'une quelconque des revendications 11 à 12, ayant un contenu en eau compris entre 8 % pds et 25 % pds.

14. Capsule selon l'une quelconque des revendications 11 à 13, comprenant en outre un revêtement en surface appliqué à l'extérieur de l'enveloppe de gélatine molle, servant à faire diminuer l'adhésivité de sa surface, et **caractérisée en ce que** le revêtement en surface comprend au moins un amidon.

15. Capsule selon la revendication 14, **caractérisée en ce que** au moins un amidon de revêtement en surface est un amidon de pomme de terre.

16. Capsule selon la revendication 14, **caractérisée en ce que** au moins un amidon de revêtement en surface est un amidon de maïs.
